# EUROPEAN PATENT APPLICATION

(11) **EP 4 400 093 A1**
(43) Date of publication of application: **17.07.2024**
(21) Application number: 23151607.1
(22) Date of filing: 13.01.2023
(51) Int. Cl.: A61K 8/9789, A61Q 19/08

(54) **COMPOSITIONS COMPRISING EPILOBIUM FLEISCHERI EXTRACT FOR TREATING SIGNS OF AGING**

(71) Applicant: DSM IP Assets B.V., 6411 TE Heerlen (NL)
(72) Inventor: BUDEL, Leithe, 4303 Kaiseraugst (CH)
(74) Representative: dsm-firmenich IP

(57) **Abstract**

The present invention relates to the (cosmetic) use of an Epilobium fleischeri extract for treating the signs of hair aging.

## Description

The present invention relates to the (cosmetic) use of an Epilobium fleischeri extract for treating the signs of hair aging.

Life expectancy is constantly increasing in many countries around the globe. Accordingly, the demand for treatments counteracting or even reversing aging processes is constantly growing, not only for age-related diseases but also for dealing with the physical signs of aging. Thus, it is not surprising that the need for cosmetic products, which are able to combat the signs of hair aging such as hair loss, has been growing significantly lately.

Equally to skin, hair is also subject to intrinsic or physiologic aging and extrinsic aging caused by external factors. Intrinsic factors are related to individual genetic and epigenetic mechanisms with interindividual variation. Prototypes are for example familial androgenetic alopecia. Extrinsic factors for hair aging include ultraviolet radiation and smoking.

Today, it is well established that cell senescence plays an important role in aging processes. Cell senescence is a physiological process and a tumour-suppressive cell fate characterized by a permanent and irreversible cell cycle arrest as well as acquisition of a pro-inflammatory and proteolytic secretome. Cells that become senescent grow in size and cease to replicate. In addition, senescent cells generate a potent mix of molecules known as the senescence-associated secretory phenotype (SASP), which provokes the immune system into an inflammatory state, disrupts tissue structure and function, and in turn encourages nearby cells to also become senescent. The SASP is one of the key characteristics that distinguish senescent cells from quiescent, terminally differentiated, and other types of non-proliferating cells.

Intrinsic aging is typically correlated with an increase of senescent cells, including senescent hair follicle cells. Senescence of hair follicle dermal papilla cells (HFDPCs) for example is considered a key driver of hair loss and baldness. Hence, there is an ongoing need to find agents which are able to selectively get rid of these errant cells.

Senolytics, which are typically understood as agents able to selectively eliminate senescent cells, were the first potential senotherapy to be successfully tested in preclinical in vivo models. In other words, an agent is considered as having senolytic activity when its activity includes specifically inducing or promoting cell death, in particular apoptotic cell death, of senescent cells in the respective tissue. Several senolytic agents have been identified by now such as navitoclax. Said senolytic agents are ultimately able to selectively eliminate senescent cells by inducing apoptosis processes that do not occur in non-senescent or normal cells, keeping these therefore unaffected.

A drawback of currently known senolytic agents is that they are often of synthetic origin, have rather complex chemical structures that require significant efforts to be obtained, exhibit low solubility in conventional cosmetic oils and/or may have severe undesired side effects.

Therefore, there is an ongoing need for senolytic agents of natural origin with reduced undesired side effects and that are obtainable in an economic manner.

Surprisingly, it has now been found that Epilobium fleischeri extract exhibits a senolytic activity on hair follicle dermal papilla cells (HFDPCs) and can thus be used to treat the signs of hair aging.

Accordingly, in a first embodiment, the present invention relates to the cosmetic use of an Epilobium fleischeri extract as an active ingredient for the prevention, reduction, or treatment of the signs of hair aging.

In another embodiment, the invention also provides the use of an Epilobium fleischeri extract as an active ingredient in, and for the manufacture of, hair care compositions for treating signs of hair aging.

In a further embodiment, the present invention also provides an Epilobium fleischeri extract as an active ingredient for use in the selective elimination of senescent hair follicle cells.

The invention also provides the use of an Epilobium fleischeri extract as an active ingredient in, and for the manufacture of, hair care compositions for the selective elimination of senescent hair follicle cells.

The invention also provides a cosmetic method for treating signs of hair aging, comprising topically applying to the hair a hair care composition containing an Epilobium fleischeri extract.

The present invention also refers to a cosmetic hair care method for selective removal of senescent hair follicle cells preferably of senescent hair follicle dermal papilla cells (HFDPCs) by applying, onto the hair in need, at least an effective amount of an extract of an Epilobium fleischeri extract; or a composition comprising at least said extract.

As used herein, the term 'cosmetic' refers to a treatment which does not cure, treat, or prevent a disease or disorder, but instead serves the maintenance of healthy and beautiful hair.

In the context of this invention, the term 'treating' denotes reducing, preventing, or eliminating.

The expression 'signs of hair aging' as used herein denotes one or more characteristics of intrinsic or chronological hair aging such as hair loss and baldness. As noted above, the process may be amplified by extrinsic factors such as exposure to sunlight, pollutants, and cigarette smoke.,

Accordingly, the term 'treating the signs of hair aging' includes the prevention, reduction or treatment of hair loss and baldness. The term also includes stimulating hair growth as well as retaining hair.

The term 'hair cells' as used herein may refer to any hair follicle resident cell type (such as hair follicle dermal papilla cells). Preferably in all embodiments of the present invention, the term refers to (human) hair follicle dermal papilla cells (HFDPCs).

In the context of this invention, the term 'senescent' refers to a state of permanent cell cycle arrest in which cells remain metabolically active and adopt characteristic phenotypic changes. The establishment of this phenotype is believed to be either the result of telomere shortening after several cell divisions (replicative senescence) or a response to stress stimuli (stress-induced senescence). One of the defining features of senescent cells is their stable cell cycle arrest. This cell cycle exit is controlled by activation of the p53/p21 and p16^{INK4a}/Rb tumour suppressor pathways. Unlike quiescent cells, senescent cells are nonresponsive to mitogenic or growth factor stimuli; thus, they are unable to re-enter the cell cycle even in advantageous growth conditions. Senescent cells are also distinct from terminally differentiated cells, which are also irreversibly withdrawn from the cell cycle. While terminal differentiation is the result of a defined developmental programme, which turns undifferentiated precursors into specialized effector cells, senescence is mainly implemented as a cellular stress response.

Senescent cells can unambiguously be identified in vitro and in vivo since they exhibit a number of characteristics that allow their explicit identification.

For example, senescent cells often appear multinucleated, large and extended, and exhibit spindle and vacuolisation features. They also display modifications in the organisation of chromatin that can help identify them. In normal cells, DNA staining reveals completely uniform colour outlines, whereas senescent cells usually show dot-like patterns, known as senescence-associated heterochromatic foci (SAHF). This phenomenon is due to intensive remodelling in the chromatin, which results in less susceptibility for digestion by nucleases. Furthermore, senescence-related chromatin remodelling leads to profound transcriptional changes. Among the assortment of upregulated genes is a prominent subset of genes that encode secreted proteins, including cytokines and chemokines with proinflammatory properties, as well as various growth factors and proteases that together alter tissue structure and function, collectively, known as SASP. The SASP is one of the key characteristics that distinguish senescent cells from quiescent, terminally differentiated, and other types of non-proliferating cells.

A distinctive measurable feature of senescent cells is the presence of β-galactosidase enzymatic activity. This enzyme normally displays activity at pH 4.0 within lysosomes, but in senescent cells it is also active at pH 6.0. This phenomenon is termed senescence associated-β-galactosidase (SA- β-gal) activity and is thought to be due to an enlargement in the structure of lysosomes in senescent cells. SA-β-gal activity is detectable by histochemical staining by using X-gal as a substrate for SA-β-gal. Since SA-β-gal activity is detected in most senescent settings, both in vitro and in vivo, it is considered a de facto hallmark of senescence. SA-β-gal can be visualized in the cells to quantify the senescence levels using flow cytometry.

The term 'senolytic activity' as used herein refers to specifically (also: selectively) inducing or promoting cell death of senescent cells. This may also be considered as ability for eliminating selectively senescent cells, for example, by inducing or promoting apoptosis. It may also be the capability to selectively eliminate senescent cells found in a tissue with a reduced or even no harmful effect on the normal resident (i.e., non-senescent) cells of the said tissue. Such senolytic property may be beneficial, for example, for preventing or attenuating the increase of the number of age-related senescent cells and/or stress-related senescent cells within a tissue.

Selective elimination of senescent cells may be determined by the change in ratio of senescent (decreasing) versus proliferating cells (increasing) based on SA-β-gal activity in the cell population model after incubation with a fixed concentration of a test compound in a suitable medium as illustrated in the examples. Such a cell population model may contain a set percentage ratio of senescent versus proliferating cells. By comparing with and without added test compound conditions, senolytic activity can be evaluated.

Compounds may be determined as capable of selectively eliminating senescent cells if the calculated senescent cell ratio (based on SA-β-gal activity) is reduced in a statistically significant manner versus the control (no compound added during incubation).

Preferably, in one embodiment the number of senescent cells is reduced by at least 20%, more preferably by at least 25%, most preferably by at least 30%, based on the initial number of senescent cells present. It is well understood, that concomitantly, the number of normal cells is increased by the same factor.

Preferably, in another embodiment, the number of senescent cells is reduced by at least a statistical difference in percentage between the control and treated conditions, more preferably by at least 50% in difference (half of original senescent cells removed), most preferably by at least 90% in difference, based on the initial number of senescent cells present. It is well understood, that concomitantly, the number of normal cells is increased by the same factor.

As used herein, the term 'extract' may be understood in the broadest sense as generally understood in the art. An extract may be any substance made by extracting a part of a raw material (here: flowers, leafs, seeds and/or stems of Epilobium fleischeri).

In a preferred embodiment, the Epilobium fleischeri extract has senolytic activity including specifically inducing or promoting cell death of senescent cells in the tissue. In this context, cell death preferably is apoptotic cell death.

Accordingly, the Epilobium fleischeri extract, according to the present invention, may be obtained by any means known to a person skilled in the art. It may optionally be obtained by a commercial supplier or may be partly or completely prepared from an Epilobium fleischeri plant or one or more parts thereof.

The extract may be any kind of extract. It may be prepared from fresh flowers, leafs, seeds and/or stems of Epilobium fleischeri or may be prepared from the dried flowers, leafs, seeds and/ or stems of Epilobium fleischeri or may be prepared from the frozen or freeze-dried flowers, leafs, seeds and/or stems of Epilobium fleischeri. Preferably, in all embodiments of the present invention, the extract is derived from the flowers and leafs or from the flowers, leafs and stems of Epilobium fleischeri. Preferably in all embodiments, the Epilobium fleischeri extract is derived from the flowers, leafs and stems.

Preferably, the flowers, leafs, seeds and/or stems of Epilobium fleischeri may be used in chopped, blended or pulverized form. In a preferred embodiment, the extract is prepared from the dried flowers, leafs, seeds and/or stems of Epilobium fleischeri, in particular from dried and pulverized flowers, leafs and/or stems of Epilobium fleischeri.

Typically, extraction is achieved by using a solvent. Accordingly, in a preferred embodiment, the extract according to the present invention is obtained from solvent extraction methods including cold or hot extraction, ultrasonic extraction, reflux cooling, needle extraction, and microwaves extraction. In a preferred embodiment, the extract is obtained by performing an ultrasonic extraction method.

One or more solvents may be used for extraction. Such solvent may be any solvent suitable for this purpose. Typically, a solvent is liquid under the conditions it is used for extraction. A solvent may be selected from the group consisting of an organic solvent (e.g., an alcohol (e.g., methanol, ethanol, propanol, butanol, pentanol, phenol, glycerol, 1 ,3-butylene glycol, propane diol, etc.), water (including hot water or subcritical water), aqueous buffer, and a combination of two or more thereof. An organic solvent may be aliphatic or aromatic. Preferably, the solvent may be hydrated.

Herein, a solvent may be added to the flowers, leafs, seeds and/or stems of Epilobium fleischeri and left together for a certain time. In a further step, the solid parts are preferably separated by the primary extract. This may be achieved by any means such as, e.g., filtration, sieving, ultrafiltration, crossflow filtration, centrifugation, precipitation over time, or a combination of two or more thereof.

Preferably, in all embodiments of the present invention the Epilobium fleischeri extract used according to the present invention is prepared by extraction with a polar solvent such as water, ethanol, glycerin, glycols or mixtures thereof, preferably with ethanol or water as well as mixtures thereof.

In all embodiments of the present invention, the flowers, leafs and/or stems of Epilobium fleischeri are preferably extracted with an ethanol/water mixture containing between 10 and 90% (m/m) ethanol, or containing between 25 and 85% (m/m) ethanol, or containing between 40 and 75% (m/m) ethanol, or containing between 50 and 70% (m/m) ethanol, or containing between 50 and 60 % (m/m) ethanol.

In all embodiments of the present invention, extraction is advantageously conducted at a temperature in the range of 10 to 150°C, or 15 to 100°C, or 20 to 80°C, or 20 to 70°C or 20 to 60°C, or approximately 20°C. In a preferred embodiment, extraction is conducted at a temperature selected in the range of ambient temperature (i.e., 15 to 25°C, in particular approximately 20°C) to 60°C.

In all embodiments of the present invention, extraction is preferably conducted at a temperature selected in the range of approximately 20°C (RT) to 60°C and ambient pressure (i.e., 980 to 1200 mbar) for 1 to 3 hours.

In all embodiments of the present invention, the Epilobium fleischeri extract can either be a liquid extract, such as an aqueous extract, or a dry extract.

As used herein, the term 'dry extract' may be understood in the broadest sense as an extract according to the present invention as dry matter, i.e., the extraction product without the solvent. The dry extract may optionally be physically present as dry matter. It will, however, be directly understood that, in calculations referring a percentage referred to dry matter, it does not have to be dry matter present in physical form, but the dry extract may also be present in dissolved form. Then, in the calculation, the mass of the solvent is mathematically subtracted from the total mass.

Dry matter (in physical form) may be obtained by a suitable extraction method, followed by a step of drying the extract obtained. The drying may be performed by any method suitable for this purpose known in the art. Drying means removing the one or more solvents used for extraction. Removing the one or more solvents may be performed by any means. For example, it may be achieved by subjecting the extract to a hot and dry atmosphere and/or placing the extract on a heated plate in order to evaporate the solvent. For example, drying may be achieved by evaporation in a vacuum and/or at elevated temperature (e.g., in a rotary (vacuum) evaporator) or by crystallization of the solid material of interest. Alternatively, or additionally, drying may be achieved by atomisation or by lyophilisation.

Preferably, in all embodiments of the present invention, the Epilobium fleischeri extract is an aqueous or a dry (in physical form) extract. An aqueous Epilobium fleischeri extract is preferably prepared by a process encompassing the steps of milling aerial parts such as in particular the flowers, leafs and stems of Epilobium fleischeri which preferably have preferably been dried beforehand, more preferably under hot air followed by extraction of the dried plants with a solvent, preferably with a mixture of ethanol/water with all the definitions and preferences as given herein, followed by removal of the ethanol e.g., by (vacuum)distillation to obtain the aqueous Epilobium fleischeri extract.

The dry extract is obtained by vacuum distillation of ethanol and water, preferably at a temperature selected in the range of 50 to 70°C until obtainment of the dry extract.

In all embodiments of the present invention said aqueous or dry extract of Epilobium fleischeri may be admixed with further ingredients such as preferably water, glycerine, citric acid and preservative(s) such as potassium sorbate, e.g., before being used to be incorporated into a hair care composition according to the present invention.

Preferably, in all embodiments according to the present invention s the Epilobium fleischeri extract to be used in the hair care compositions as disclosed herein contains (i) from 5 to 10 wt.-% based on dry matter of the Epilobium fleischeri flower, leaf stem extract, (ii) from 25 to 50 wt.-% of water, (iii) > 50 wt.-% of a diol such as preferably glycerine, and (iv) from 0.1 to 1 wt.-% of one or more additives such as preferably citric acid and/or potassium sorbate while advantageously, all ingredients sum up to 100 wt.-%.

In all embodiments of the present invention the Epilobium fleischeri extract is preferably administered in the form of a hair care composition comprising an effective amount of the Epilobium fleischeri extract and a cosmetically acceptable carrier.

In all embodiments of the present invention, the hair care compositions are preferably applied topically, such as to the hair and here in particular to the scalp.

The term 'an effective amount' refers to an amount necessary to obtain the desired physiological effect. The physiological effect may be achieved by one application dose or by repeated applications. The dosage administered may, of course, vary depending upon known factors, such as the physiological characteristics of the particular composition comprising the Epilobium fleischeri extract and its mode and route of administration; the age, health and weight of the recipient; the kind of concurrent treatment; the frequency of treatment; and the effect desired and can be adjusted by a person skilled in the art.

The term 'cosmetically acceptable carrier' (also referred to herein as carrier) refers to all vehicles/carriers conventionally used in hair care composition, i.e., which are suitable for topical application to the keratinous tissue, have good aesthetic properties, are compatible with the actives present in the composition, and will not cause any unreasonable safety or toxicity concerns. Such carriers are well-known to one of ordinary skill in the art and can include one or more compatible liquid(s) or solid filler diluent(s), excipient(s), additive(s) or vehicle(s) which are suitable for application to hair.

Generally, the amount of the Epilobium fleischeri extract in the hair care compositions according to the present invention is selected in the range from 0.001 to 10 wt.-%, more preferably from 0.01 to 8 wt.-%, even more preferably from 0.1 to 7 wt.-%, furthermore preferably from 0.1 to 5 wt.-% and still more preferably from 0.1 to 3 wt.-%, based on the total weight of the hair care composition. Further suitable ranges are from 0.05 to 2 wt.-% and from 0.1 to 1 wt.-%.

The exact amount of carrier will depend upon the actual level of the Epilobium fleischeri extract and of any other optional ingredients that one of ordinary skill in the art would classify as distinct from the carrier (e.g., other active ingredients)

In an advantageous embodiment, the hair care compositions according to the present invention comprise from about 50% to about 99%, preferably from about 60% to about 98%, more preferably from about 70% to about 98%, such as in particular from about 80% to about 95% of a carrier, based on the total weight of the hair care composition.

In a particular advantageous embodiment, the carrier consists furthermore of at least 40 wt.-%, more preferably of at least 50 wt.-%, most preferably of at least 55 wt.-% of water, such as in particular of about 55 to about 90 wt.-% of water.

The hair care compositions according to the present invention are preferably prepared by admixing the Epilobium fleischeri extract with all the definitions and preferences as given herein with/into a cosmetically acceptable carrier.

The hair care composition may comprise further ingredients, which may form part of the carrier. Such ingredients are particularly surfactants, emulsifiers, thickeners, and oils. Such suitable surfactants, emulsifiers, thickeners, and oils are well known to a person skilled in the art.

The hair care compositions according to the present invention (including the carrier) may comprise further conventional (cosmetic) adjuvants and additives, such as preservatives/antioxidants, fatty substances/oils, water, organic solvents, silicones, thickeners, softeners, emulsifiers, antifoaming agents, aesthetic components such as fragrances, surfactants, fillers, anionic, cationic, non-ionic or amphoteric polymers or mixtures thereof, propellants, acidifying or basifying agents, dyes, colourings/colorants, abrasives, absorbents, chelating agents and/or sequestering agents, essential oils, hair sensates, astringents, pigments or any other ingredients usually formulated into such compositions.

In accordance with the present invention, the compositions according to the invention may also comprise further cosmetically active ingredients conventionally used in topical hair care composition. Exemplary active ingredients encompass UV-filters, cleansing agents, conditioning agents, antimicrobial and/ or antifungal agents; moisturizing, soothing, and/or energizing agents.

If nothing else is stated, the excipients, additives, diluents, etc. mentioned in the following are suitable for the compositions according to the present invention. The necessary amounts of the cosmetic and dermatological adjuvants and additives can, based on the desired product, easily be determined by the skilled person.

The additional ingredients can either be added to the oily phase, the aqueous phase or separately as deemed appropriate. The mode of addition can easily be adapted by a person skilled in the art.

Examples of cosmetic excipients, diluents, adjuvants, additives as well as active ingredients commonly used in the hair care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The final product form of the hair care compositions according to the invention may suitably be, for example, shampoos, conditioners, sprays, mousses, gels, oils, creams, waxes or lotions. Particularly preferred product forms are leave-in products, especially post-wash conditioners (leave-in) and hair treatment products such as hair essences.

Conditioner compositions according to the present invention usually comprise one or more conditioning surfactants, which are cosmetically acceptable and suitable for topical application to the hair. Suitable conditioning surfactants are selected from cationic surfactants, used singly or in a mixture.

Cationic surfactants useful in compositions according to the present invention contain amino or quaternary ammonium hydrophilic moieties, which are positively charged when, dissolved in the aqueous composition of the present invention.

The most preferred cationic surfactants for conditioner compositions according to the present invention are monoalkyl quaternary ammonium compounds in which the alkyl chain length is C₁₆ to C₂₂.

Examples of suitable cationic surfactants include quaternary ammonium compounds, particularly trimethyl quaternary compounds. Preferred quaternary ammonium compounds include cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these where the chloride is replaced by halogen, (e.g., bromide), acetate, citrate, lactate, glycolate, phosphate nitrate, sulphate, or alkylsulphate. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners according to the present invention is cetyltrimethylammonium chloride.

Salts of primary, secondary, and tertiary fatty amines are also suitable cationic surfactants.

In the conditioners according to the present invention, the level of cationic surfactant is preferably selected in the range from 0.01 to 10 wt.-%, more preferably from 0.05 to 5 wt.-%, most preferably from 0.1 to 2 wt.-%, based on the total weight of the composition.

Conditioner compositions according to the present invention preferably additionally comprise fatty materials. By "fatty material" is meant a fatty alcohol, an alkoxylated fatty alcohol, a fatty acid, a glyceride, glycerol, plant unsaponifiables or a mixture thereof. Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of the composition.

Ethoxylated fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty alcohol material in conditioners according to the present invention is suitably selected in the range from 0.01 to 15 wt.-%, preferably from 0.1 to 10 wt.-%, and more preferably from 0.1 to 5 wt.-%, based on the total weight of the composition. The weight ratio of cationic surfactant to fatty alcohol is preferably selected in the range from 10:1 to 1:10, preferably from 4:1 to 1:8, most preferably from 1:1 to 1:7, such as for example 1:3.

In a preferred embodiment, the hair care compositions, especially if it is a shampoo, further comprises from 0.1 to 5 wt.-%, based on the total weight of the composition of a suspending agent.

Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives, and mixtures thereof. The long chain acyl derivative is desirably selected from ethylene glycol stearate, alkanolamides of fatty acids having from 16 to 22 carbon atoms and mixtures thereof. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich. Suitable cross-linked polymers of acrylic acid and acrylate esters are Pemulen TR1 or Pemulen TR2 (both available from Lubrizol). A suitable heteropolysaccharide gum is xanthan gum.

The hair care compositions according to the present invention may further contain emulsified droplets of a silicone-conditioning agent for enhancing conditioning performance. Suitable silicones include polydiorganosiloxanes, in particular polydimethylsiloxanes which have the CTFA designation dimethicone. Also suitable for use compositions according to the present invention (particularly shampoos and conditioners) are polydimethyl siloxanes having hydroxyl end groups, which have the CTFA designation dimethiconol. Also suitable for use in compositions according to the present invention are silicone gums having a slight degree of cross-linking.

Examples of suitable pre-formed silicone emulsions include dimethiconol emulsions DC2-1766, DC2-1784, DC-1785, DC-1786 and DC-1788, all available from Dow Corning. Also suitable are amodimethicone emulsions such as DC2-8177 and DC939 (from Dow Corning) and SME253 (from GE Silicones).

A further preferred class of silicones for inclusion in shampoos and conditioners of the present invention have at least one amino functional group.

The total amount of silicone is preferablyselected in the range from 0.01 to 10 wt.-%, more preferably from 0.3 to 5 wt.-%, most preferably 0.5 to 3 wt.-%, based on the total weight of the composition.

The hair care compositions according to the present invention may also comprise a dispersed, non-volatile, water-insoluble oily conditioning agent.

By "insoluble" is meant that the material is not soluble in water (distilled or equivalent) at a concentration of 0.1 percent w/w at 25 degrees centigrade Suitable oily or fatty materials are selected from hydrocarbon oils, fatty esters and mixtures thereof. Adjuvants

The hair care compositions of the present invention may also contain adjuvants suitable for hair care. Generally, such ingredients are included individually at a level of up to 2 wt.-%, preferably up to 1 wt.-%, based on the total weight of the composition.

Suitable hair care adjuvants, include amino acids, sugars and ceramides. Particularly preferred are anti-dandruff agents, especially those comprising zinc, such as zinc pyrithione (ZnPTO). A further preferred ingredient is climbazole.

The hair styling polymer, if present, is preferably present in the hair care compositions according to the present invention in an amount of from 0.001 to 10 wt.-%, more preferably from 0.1 to 10 wt.-%, such as from 1 to 8 wt.-%, based on the total weight of the composition. Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

Shampoo compositions preferably comprise one or more cleansing surfactants, which are cosmetically acceptable and suitable for topical application to the hair. Further surfactants may be present as emulsifiers.

Suitable cleansing surfactants are selected from anionic, amphoteric and zwitterionic surfactants and mixtures thereof. The cleansing surfactant may be the same surfactant as the emulsifier or may be different. Anionic Cleansing Surfactant

Shampoo compositions according to the invention will typically comprise one or more anionic cleansing surfactants, which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates and alpha-olefin sulphonates, especially their sodium, magnesium, ammonium and mono-, di- and triethanolamine salts.

Typical anionic cleansing surfactants for use in shampoo compositions according to the present invention include sodium oleyl sulpho succinate, ammonium lauryl sulphosuccinate, ammonium lauryl sulphate, sodium cocoyl isethionate, sodium lauryl isethionate and sodium N-lauryl sarcosinate. The most preferred anionic surfactants are sodium lauryl sulphate, sodium lauryl ether sulphate (n)EO (where n ranges from 1 to 3), ammonium lauryl sulphate and ammonium lauryl ether sulphate (n)EO (where n ranges from 1 to 3).

The total amount of anionic cleansing surfactant in shampoo compositions according to the present invention is generally from 5 to 30 wt.-%, preferably from 6 to 20 wt.-%, more preferably from 8 to 16 wt.-%, based on the total weight of the composition.

The shampoo composition may also include co-surfactants, preferably an amphoteric or zwitterionic surfactant, which can be included in an amount ranging from 0.01 to about 8 wt.-%, preferably from 1 to 4 wt.-%, based on the total weight of the composition.

Examples of amphoteric and zwitterionic surfactants include, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoo compositions according to the present invention include lauryl amine oxide, cocodi methyl sulphopropyl betaine and preferably lauryl betaine, cocamidopropyl betaine and sodium cocamphopropionate.

Another preferred co-surfactant is a nonionic surfactant, which can be included in an amount ranging from 0.01 to 8 wt.-%, preferably from 2 to 5 wt.-%, based on the total weight of the composition.

For example, representative nonionic surfactants that can be included in shampoo compositions according to the present invention include condensation products of aliphatic (C₈ - C₁₈) primary or secondary linear or branched chain alcohols or phenols with alkylene oxides, usually ethylene oxide and generally having from 6 to 30 ethylene oxide groups.

Further nonionic surfactants which can be included in shampoo compositions according to the present invention are the alkyl polyglycosides (APGs). Such APG's are well known to a person skilled in the art and e.g. commercially available as Plantacare UP 2000.

The shampoo composition can also optionally include one or more cationic co- surfactants included in an amount ranging from 0.01 to 10 wt.-%, more preferably from 0.05 to 5 wt.-%, most preferably from 0.05 to 2 wt.-%, based on the total weight of the composition. Useful cationic surfactants are described hereinbelow in relation to conditioner compositions.

The total amount of surfactant (including any co-surfactant, and/or any emulsifier) in shampoo compositions according to the present invention is generally from 5 to 50 wt.-%, preferably from 5 to 30 wt.-%, more preferably from 10 to 25 wt.-%, based on the total weight of the composition.

A cationic surfactant is a preferred ingredient in compositions according to the present invention for enhancing conditioning performance. Suitable cationic conditioning surfactants include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (e.g., Arquad 2HT/75 from Akzo Nobel), cocotrimethylammonium chloride, PEG-2-oleammonium chloride and the corresponding hydroxides thereof, Quaternium-5, Quaternium-31, Quaternium-18 and mixtures thereof.

Another example of a class of suitable cationic conditioning surfactants either alone or in admixture with one or more other cationic conditioning surfactants, is a combination of an amidoamine and an acid. Preferred amidoamines useful herein include stearamido-propyldimethylamine, stearamidopropyldiethylamine, stearamidoethyldiethylamine, stearamidoethyldimethylamine, palmitamidopropyldimethylamine, palmitamido-propyldiethylamine, palmitamidoethyldiethylamine, palmitamidoethyldimethylamine, behenamidopropyldimethylamine, behenamidopropyldiethylmine, behenamido-ethyl-diethylamine, behenamidoethyldimethylamine, arachidamidopropyldimethylamine, arachidamidopropyldiethylamine, arachid- amidoethyldiethylamine, arachidamido-ethyldimethylamine, and mixtures thereof. The acid may be any organic or mineral acid which is capable of protonating the amidoamine in the hair care composition thus forming a tertiary amine salt which is in effect is a non-permanent quaternary ammonium or pseudo-quaternary ammonium cationic surfactant. Suitable acids useful herein include hydrochloric acid, acetic acid, tartaric acid, fumaric acid, lactic acid, malic acid, succinic acid, and mixtures thereof. Suitably, the acid is included in a sufficient amount to protonate all the amidoamine present.

Cationic polymers are preferred ingredients for enhancing conditioning performance. Suitable cationic polymers may be homopolymers which are cationically substituted or may be formed from two or more types of monomers. The weight average (Mw) molecular weight of the polymers will generally be between 100 000 and 2 million Daltons. Cationic polymer will generally be present in a shampoo composition at levels of from 0.01 to 5 wt.-%, preferably from 0.05 to 1 wt.-%, more preferably from 0.08 to 0.5 wt.-%, based on the total weight of the composition. Suitable cationic polymers include, for example, cationic polysaccharide polymers, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C₁-C₇ alkyl groups, more preferably C₁-₃ alkyl groups. Other suitable spacers include vinyl esters, vinyl alcohol, maleic anhydride, propylene glycol and ethylene glycol.

The cationic surfactant or polymer will generally be present in compositions according to the present invention at levels of from 0.01 to 5 wt.-%, preferably from 0.05 to 1 wt.-%, more preferably from 0.08 to 0.5 wt.-%, based on the total weight of the composition. The combined use of fatty alcohols and cationic surfactants in conditioning compositions is believed to be especially advantageous, because this leads to the formation of a lamellar phase, in which the cationic surfactant is dispersed. The weight ratio of cationic surfactant to fatty alcohol is suitably selected in the range from 1:1 to 1:10, preferably from 1:1.5 to 1:8, most preferably from 1:2 to 1:5. If the weight ratio of cationic surfactant to fatty alcohol is too high, this can lead to eye irritancy from the composition. If it is too low, it can make the hair feel squeaky for some consumers.

the hair care compositions according to the present invention may contain other ingredients for enhancing performance and/or consumer acceptability. Such ingredients include fragrance, dyes and pigments, pH adjusting agents, pearlescence agents or opacifiers, viscosity modifiers, and preservatives or antimicrobials. Each of these ingredients will be present in an amount effective to accomplish its purpose. Generally, these optional ingredients are included individually at a level of up to 5 wt.-%, based on the total weight of the composition.

Hair care compositions according to the invention are produced using methods known to the person skilled in the art.

The following examples are provided to further illustrate the compositions and effects of the present invention. These examples are illustrative only and are not intended to limit the scope according to the present invention in any way.

### Examples

### 1. Preparation of dry plant extracts

Dried aerial parts (preferably flowers and/or leaves) of the respective plants are crushed and then extracted with a solvent (mixture of ethanol/water from 50% ethanol/50% water (m/m) to 60% ethanol/40% water) at a temperature selected in the range of RT to 60°C for one to 3 hours. After filtration the hydro-alcoholic extracts were collected and concentrated under vacuum at a temperature between 50 and 70°C until obtention of the dry extracts.

### 2. Cultivation of senescent cells

Normal and senescent cell populations were obtained from normal hair follicle dermal papilla cells. For the normal cell population, the cells were seeded in cell culture medium (Dulbecco's Modified Eagle Medium supplemented with 10% serum and 1% antibiotics) and allowed to adhere and proliferate in a humidified cell incubator maintaining 37°C and 5% CO₂. These were then passaged or collected when 90% confluent. For the senescent cell population, the normal fibroblast cells were at this stage shortly exposed to 200 µM H₂O₂ followed by normal cell culture medium. Several days later, the cells were treated again with H₂O₂ to obtain a fully senescent population.

### 3. Senolytic activity study

Both normal and senescent populations were mixed into a 70% normal and 30% senescent cell population and represented the treatment population. Afterward, the 70% normal and 30% senescent cell population were seeded in cell culture medium and allowed to adhere in the cell incubator. After adherence, the medium was aspirated and the treatment medium (Dulbecco's Modified Eagle Medium supplemented with 1% serum) containing the respective dry plant extracts at various concentrations (see Table 1) was added to the cells. For controls, there was one condition with treatment medium only without compound (negative control) and one condition with navitoclax, which is a known senolytic (positive control). The cells were incubated in the cell incubator for at least 72 hours. Afterward, senescence-associated beta-galactosidase was visualized - a well-known senescence marker - in the cells to quantify the senescence levels using flow cytometry. The results are depicted in table 1.

### Results

Experimentation showed that the plant extract from Epilobium fleischeri but not from Artemisia umbelliformis, Leontopodium alpinum, Eryngium alpinum and Asparagus officinalis, exhibited a significant senolytic effect and reduced the senescence percentage ratio for human dermal fibroblasts, see Table 1.

**Table 1**

| Bioactive | Concentration | Senescent cells (%) | Standard deviation (%) | P-value | Significant senolytic effect |
|---|---|---|---|---|---|
| CTRL | | 30 | 3 | - | No |
| Navitoclax (positive control) | 3 µM | 22 | 2 | 0.004 | Yes |
| Artemisia umbelliformis (Reference) | 0.0025% | 29 | 2 | 0.417 | No |
| | 0.0005% | 32 | 1 | <0.01 | No |
| | 0.0001 % | 27 | 1 | 0.454 | No |
| Leontopodium alpinum (Reference) | 0.0025% | 27 | 2 | 0.568 | No |
| | 0.0005% | 31 | 3 | 0.244 | No |
| | 0.0001 % | 31 | 2 | 0.201 | No |
| Eryngium alpinum (Reference) | 0.0025% | 26 | 2 | 0.979 | No |
| | 0.0005% | 29 | 2 | 0.098 | No |
| | 0.0001% | 31 | 2 | <0.01 | No |
| Asparagus officinalis (Reference) | 0.0025% | 32 | 3 | 0.071 | No |
| | 0.0005% | 36 | 1 | <0.001 | No |
| | 0.0001 % | 33 | 2 | <0.05 | No |
| Epilobium fleischeri (Invention) | 0.0025% | 11 | 0 | <0.001 | Yes |
| | 0.0005% | 17 | 1 | <0.001 | Yes |
| | 0.0001% | 24 | 1 | <0.001 | Yes |

Table 1, 70% normal and 30% senescent hair follicle dermal papilla cells were treated for 72 hours with several concentrations of specific plant extracts, which resulted in the reduction of senescent cells for certain plant species. Data are normalized to the untreated control (30% senescent cells and 70% normal cells) and expressed as the mean ± SD (n = 4).

## Claims

1. Cosmetic use of an Epilobrium fleicheri extract as an active ingredient for the treatment of the signs of hair aging by selectively eliminating senescent hair cells.

2. The use according to claim 1, wherein the senescent hair cells are hair follicle dermal papilla cells.

3. The use according to claim 1 and/or 2, wherein the senescent cells are age-related senescent hair cells, stress-related senescent hair cells, or both.

4. The use according to anyone or more of claims 1 to 3, wherein the number of senescent cells is reduced by at least 20%, preferably by at least 25%, most preferably by at least 30%.

5. The use according to anyone or more of claims 1 to 4, wherein the Epilobium fleischeri extract is an aqueous or a dry extract obtained from the flowers, leafs, seeds and/or stems of the plant Epilobium fleischeri.

6. The use according to claim 5, wherein the Epilobium fleischeri extract consists essentially of (i) from 5 to 10 wt.-% based on dry matter of the Epilobium fleischeri flower, leaf and/ or stem extract, (ii) from 25 to 50 wt.-% of water, (iii) > 50 wt.-% of glycerine, and (iv) from 0.1 to 2 wt.-% of citric acid and/or potassium sorbate.

7. The use according to anyone or more of claims 1 to 6, wherein the Epilobium fleischeri extract is administered in the form of a hair care composition comprising an effective amount of Epilobium fleischeri extract and a cosmetically acceptable carrier.

8. The use according to claim 7, wherein the carrier consists of at least 30 wt. %, more preferably of at least 40 wt.-%, most preferably of at least 45 wt.-% of water, such as in particular of 50 to 90 wt.-% of water.

9. The use according to claim 7 and/or 8, wherein the hair care composition comprises from 0.001 to 10 wt.-%, preferably from 0.01 to 8 wt.-%, even more preferably from 0.1 to 7 wt.-%, furthermore preferably from 0.1 to 5 wt.-% and still more preferably from 0.1 to 3 wt.-%, of the Epilobium fleischeri extract, based on the total weight of the hair care composition.

10. The use according to anyone or more of claims 7 to 9, wherein the hair care composition is a leave-on or rinse-off composition, preferably a leave-on composition.

11. The use according to claim 10, wherein, the composition is selected from the group consisting of post-wash conditioners (leave-in) and hair treatment products such as hair essences.

12. A cosmetic hair care method for selective removal of senescent cells in hair follicles, said method encompassing the step of applying, onto the scalp in need, at least an effective amount of an extract of an Epilobium fleischeri extract; or a composition comprising at least said extract.

13. The method according to claim 12, wherein the senescent cells are senescent human hair follicle dermal papilla cells.

14. The method according to claim 12 and/or 13, wherein the effective amount of the Epilobium fleischeri extract (based on dry matter) applied to the hair is selected in the range of 0.001 to 1 wt.-%/cm² of hair.

15. The method according to anyone of claims 12 to 14, wherein the number of senescent cells is reduced by at least 20%, preferably by at least 25%, most preferably by at least 30%.
